# FASCICULE DE BREVET EUROPEEN

(11) **EP 0 476 708 B1**
(45) Date de publication et mention de la délivrance du brevet: **08.11.1995**
(21) Numéro de dépôt: 91117775.6
(22) Date de dépôt: 30.03.1989
(51) Int. Cl.: C07D 213/647, A01N 53/00, A23K 1/16, C07C 255/39, C07F 7/08, C07C 69/753

(54) **Dérivés pyréthrinoides trifluorométhyl vinyliques, leur procédé de préparation, les intermédiaires de ce procédé et leur application à titre de pesticides**
(Trifluormethyl)vinylpyrethroidderivate, ihre Verwendung als Pestizide, Verfahren zu ihrer Herstellung und Zwischenprodukte dieses Verfahrens
(Trifluoromethyl)vinyl pyrethroid derivatives, their use as pesticides, process for their preparation, and intermediates of that process

(30) Priorité: 31.03.1988 FR 8804260
(43) Date de publication de la demande: 25.03.1992
(62) Demande divisionnaire de: 89400887.9
(73) Titulaire: ROUSSEL UCLAF, 93230 Romainville (FR)
(72) Inventeur: Bonin, Werner, W-6233 Kelkheim (DE); Demoute, Jean-Pierre, F-93360 Neuilly Plaisance (FR); Tessier, Jean, F-94300 Vincennes (FR)
(74) Mandataire: Vieillefosse, Jean-Claude

(56) Documents cités:
- EP-A- 0 019 787
- EP-A- 0 061 114

## Description

La présente invention concerne des dérivés pyréthrinoïdes trifluorométhylvinyliques, leur procédé de préparation, les intermédiaires de ce procédé et leur application à titre de pesticides. EP-A-0 061 114 et EP-A-0 019 787 décrivent aussi des dérivés pyréthrinoïdes trifluorométhylviniliques qui ont des propriétés pesticides.

L'invention a pour objet des produits de formule (A) : 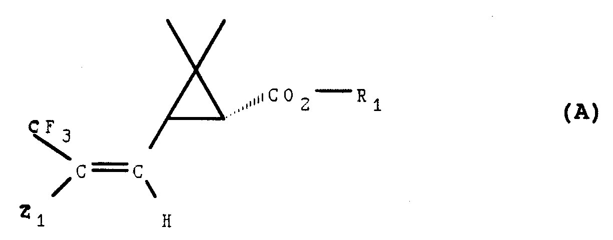
dans laquelle la copule cyclopropanique est de structure (1R, trans), la géométrie de la double liaison est Z,
Z₁ représente un radical aryle éventuellement substitué par un atome d'halogène, R₁ représente un des groupes : 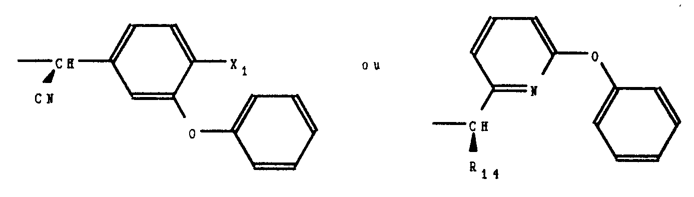
dans lesquels X₁ représente un atome d'hydrogène ou de fluor et R₁₄ représente un atome d'hydrogène ou un des radicaux méthyle, éthynyle ou cyano, notamment ceux de formule (A) dans laquelle Z₁ représente le radical 4-chloro phényle et tout particulièrement le produit de formule (A₁) : 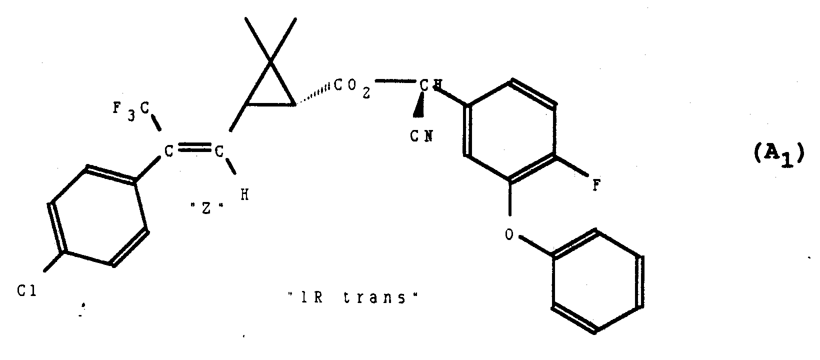
et le produit de formule (B₁) : 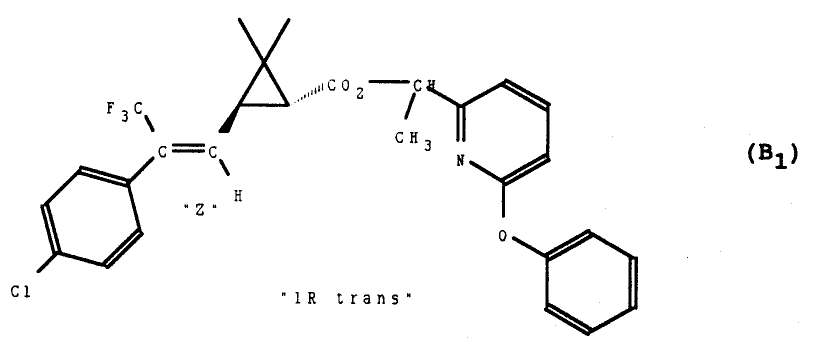
L'invention a également pour objet le procédé de préparation des produits des exemples 1 à 3. On soumet un produit de formule (II) : 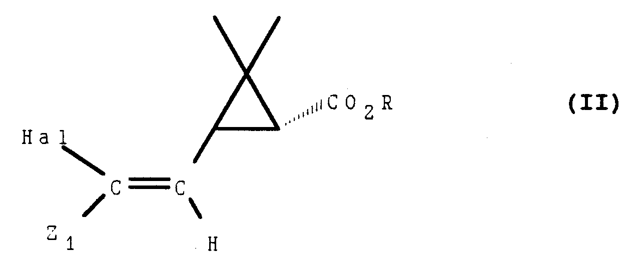
dans laquelle Hal représente un atome de brome, de chlore ou d'iode, R représente un radical alkyle renfermant jusqu'à 8 atomes de carbone, éventuellement substitué par un radical trialkylsilyle ou un radical benzyle et Z₁ est tel que défini à la revendication 1, à l'action du trifluoroacétate de sodium en présence d'iodure cuivreux pour obtenir un produit de formule (I) : 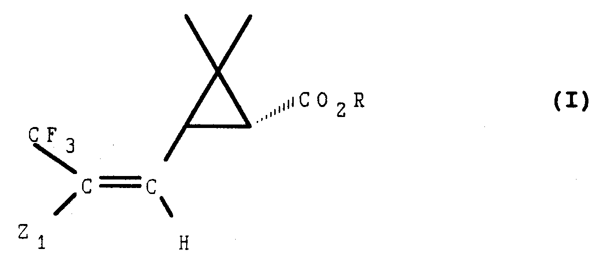
produit de formule (I) qui est transformé, comme illustré dans les exemples 1, 2 ou 3, en produit de formule (A).

Les produits de formule (I) intermédiaires du procédé dont les noms suivent :
- le [1R-(1alpha,3béta)]-3-[(Z)-2-(4-chlorophényl) 3,3,3-trifluoro 1-propényl] 2,2-diméthyl cyclopropanecarboxylate de méthyle,
- le (1R-(1alpha,3béta)]-3-[(Z)-2-(4-chlorophényl) 3,3,3-trifluoro 1-propényl] 2,2-diméthyl cyclopropanecarboxylate de 2-(triméthylsilyl) éthyle,
sont aussi objet de la présente invention.

Les composés de formule (II) sont synthétisés selon les méthodes développées dans les paragraphes ultérieurs intitulés préparation 1 et 2.

Les produits de formule (A) telle que définie précédemment possèdent d'intéressantes propriétés pesticides qui permettent leur utilisation dans la lutte contre les parasites ; il peut s'agir par exemple de la lutte contre les parasites des végétaux, les parasites des locaux et les parasites des animaux à sang chaud. C'est ainsi que l'on peut utiliser les produits de l'invention pour lutter contre les insectes, les nématodes et les acariens parasites des végétaux et des animaux.

L'invention a donc pour objet ces composés pour leur utilisation à la lutte contre les parasites des végétaux, les parasites des locaux et les parasites des animaux à sang chaud.

Ces produits peuvent donc être utilisés notamment pour lutter contre les insectes dans le domaine agricole, pour lutter par exemple contre les pucerons, les larves de lépidoptères et les coléoptères. Ils sont utilisés à des doses comprises entre 10 g et 300 g de matière active à l'hectare.

Ces produits peuvent également être utilisés pour lutter contre les insectes dans les locaux, pour lutter notamment contre les mouches, les moustiques et les blattes.

Ces produits peuvent aussi être utilisés pour lutter contre les acariens parasites des végétaux.

Ces produits peuvent aussi être utilisés pour lutter contre les nématodes parasites des végétaux.

Les composés peuvent encore être utilisés pour lutter contre les acariens parasites des animaux, pour lutter par exemple contre les tiques et notamment les tiques de l'espèce Boophilus, ceux de l'espèce Hyalomnia, ceux de l'espèce Amblyomnia et ceux de l'espèce Rhipicephalus ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits définis ci-dessus.

L'invention a notamment pour objet les compositions insecticides renfermant comme principe actif au moins l'un des produits définis ci-dessus.

Les compositions selon l'invention sont préparées selon les procédés usuels de l'industrie agrochimique ou de l'industrie vétérinaire ou de l'industrie des produits destinés à la nutrition animale.

Ces compositions peuvent se présenter sous forme de poudres, granulés, suspensions, émulsions, solutions, solutions pour aérosols, bandes combustibles, appâts ou autres préparations employés classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut-être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un électromosquito destroyer.

Dans le cas où l'on utilise un serpentin insecticide, le support inerte peut être, par exemple, composé de marc de pyrèthre, poudre de Tabu (ou poudre de feuilles de Machilus Thumbergii), poudre de tige de pyrèthre, poudre de feuille de cèdre, poudre de bois (telle que la sciure de pin) amidon et poudre de coque de noix de coco. La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

L'invention a également pour objet, les compositions acaricides renfermant comme principe actif au moins un des produits de formule (I) définie ci-dessus.

L'invention a également pour objet les compositions nématicides renfermant comme principe actif au moins un des produits de formule (I) ci-dessus.

Les compositions insecticides selon l'invention, comme les compositions acaricides et nématicides peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % ou des liquides pour pulvérisation foliaire contenant de 1 à 500 g/l de principe actif. On peut également employer des poudres pour poudrage foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

L'invention a également pour objet les compositions acaricides destinées à la lutte contre les acariens parasites des animaux à sang chaud, notamment contre les tiques et les gales, caractérisées en ce qu'elles renferment comme principe actif, au moins l'un des produits de formule (I) définie ci-dessus.

Pour exalter l'activité biologique des produits de l'invention, on peut les additionner à des synergistes classiques utilisés en pareil cas tel le 1-(2,5,8-trioxododécyl) 2-propyl 4,5-méthylènedioxy benzène (ou butoxyde de pipéronyle) ou la N-(2-éthyl heptyl) 5-bicyclo[2,2-1]heptène 2,3-dicarboximide, ou le pipéronyl-bis-2(2'-n-butoxy éthoxy) éthylacétal (ou tropital).

Lorsqu'il s'agit de lutter contre les acariens parasites des animaux, on incorpore très souvent les produits de l'invention dans des compositions alimentairs en association avec un mélange nutritif adapté à l'alimentation animale. Le mélange nutritiel peut varier selon l'espèce animale, il peut renfermer des céréales, des sucres et des grains, des tourteaux de soja, d'arachide et de tournesol, des farines d'origine animale, par exemple des farines de poissons, des acides aminés de synthèse, des sels minéraux, des vitamines et des anti-oxydants. L'invention a donc également pour objet les compositions destinées à l'alimentation animale, renfermant comme principe actif au moins l'un des produits de formule (I) telle que définie précédemment.

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisis dans le groupe constitué par les esters d'alléthrolone, d'alcool 3,4,5,6-tétrahydrophtalimido méthylique, d'alcool (5-benzyl 3-furyl) méthylique, d'alcools 3-phénoxy benzylique et d'alcools alpha-cyano 3-phénoxy benzyliques des acides chrysanthémiques, par les esters d'alcools (5-benzyl 3-furyl) méthylique des acides 2,2-diméthyl 3-(2-oxo 3-tétrahydrothiophénylidène méthyl) cyclopropanecarboxyliques, par les esters d'alcool 3-phénoxy benzylique et d'alcools alpha-cyano 3-phénoxy benzyliques des acides 2,2-diméthyl 3-(2,2-dichlorovinyl) cyclopropanecarboxyliques, par les esters d'alcool alpha-cyano 3-phénoxy benzyliques d'acides 2,2-diméthyl 3-(2,2-dibromovinyl) cyclopropanecarboxyliques, par les esters d'alcool 3-phénoxy benzylique des acides 2-parachlorophényl 2-isopropyl acétiques, par les esters d'alléthrolones d'alcool 3,4,5,6-tétrahydrophtalimidométhylique, d'alcool (5-benzyl 3-furyl) méthylique, d'alcool 3-phénoxy benzylique et d'alcools alpha-cyano 3-phénoxy benzyliques des acides 2,2-diméthyl 3-(1,2,2,2-tétrahaloéthyl) cyclopropanecarboxyliques, dans lesquels "halo" représente un atome de fluor, de chlore ou de brome.

Les associations selon l'invention présentent notamment l'intérêt soit de permettre de combattre, par la polyvalence de leur action, une gamme de parasites, plus étendue, soit de manifester, dans certains cas, un effet de synergie.

L'invention a pour objet les compositions pesticides définies précédemment, caractérisées en ce qu'elles renferment en outre un synergiste des pyréthrinoïdes.

Comme synergistes classiques utilisés en pareil cas, on peut citer le 1-(2,5,8-trioxadodécyl) 2-propyl 4,5-méthylènedioxy benzène (ou butoxyde de pipéronyle) ou le N-(2-éthyl heptyl) bicyclo[2,2-5]heptène 2,3-dicarboximide, ou le pipéronyl-bis-2-(2'-n-butoxy éthoxy) éthyl acétal (tropital).

Les exemples suivants illustrent l'invention.

### EXEMPLE 1 : [1R-[1alpha(R*),3béta]]-2,2-diméthyl 3-[(Z)-2-(4-chlorophényl) 3,3,3-trifluoro 1-propényl] cyclopropanecarboxylate de 1-(6-phénoxy 2-pyridyl) éthyle.

### STADE A : [1R-(1alpha,3béta)]-3-[(Z)-2-(4-chlorophényl) 3,3,3-trifluoro 1-propényl] 2,2-diméthyl cyclopropanecarboxylate de méthyle

On dissout 2,95 g de [1R-(1alpha,3béta)]-3-[(Z)-2-bromo 2-(4-chlorophényl) éthényl] 2,2-diméthyl cyclopropanecarboxylate de méthyle (préparation 1) et 4,68 g de trifluoroacétate de sodium dans 30 cm³ de N-méthyl pyrrolidone. On ajoute 3,27 g d'iodure de cuivre. On chauffe pendant 6 heures à 160°C environ. On laisse revenir à la température ambiante, filtre, rince et distille. On obtient un résidu que l'on extrait à l'éther isopropylique. On lave, sèche et amène à sec. On obtient 3,94 g de produit que l'on chromatographie sur silice en éluant par le mélange hexane-éther isopropylique (95-5). On obtient 2,22 g de produit recherché.

| Spectre RMN CDCl₃ : | |
|---|---|
| protons de méthyles géminés | 1,25 - 1,35 ppm |
| protons aromatiques | 7,13 7,5 ppm |
| proton éthylénique | 5,63 5,80 ppm |
| protons du carbone en 3 du cyclopropane | 2,33 - 2,75 ppm |
| protons du carbone en 1 du cyclopropane | 1,69 - 1,78 ppm |
| protons de CO₂-CH₃ | 3,75 ppm. |

### STADE B : [1R-[1alpha(R*),3béta]]-2,2-diméthyl 3-[(Z)-2-(4-chlorophényl) 3,3,3-trifluoro 1-propényl] cyclopropanecarboxylate de 1-(6-phénoxy 2-pyridyl) éthyle

On dissout 0,85 g de produit préparé au stade A dans 3,75 cm³ d'une solution normale de soude. On agite la solution obtenue pendant 7 heures à 40°C. On verse dans un mélange d'eau et de glace, ajoute 4 cm³ d'une solution normale d'acide chlorhydrique, lave à l'eau, à l'aide d'une solution saturée de chlorure de sodium, sèche et amène à sec. On obtient 0,84 g de produit que l'on dissout, avec 540 mg de (R)-6-(3-phénoxy) alpha-méthyl 2-pyridine méthanol, dans 4 cm³ de chlorure de méthylène. On refroidit au bain de glace et ajoute une solution renfermant 520 mg de dicyclohexylcarbodiimide, 8 mg de 4-diméthylaminopyridine et 3 cm³ de chlorure de méthylène. On agite 5 minutes au bain de glace et une nuit à la température ambiante. On filtre, rince à l'éther isopropylique et amène à sec. On récupère 1,38 g de produit que l'on chromatographie sur silice en éluant avec le mélange hexane-éther isopropylique (8-2). On obtient 1 g du produit recherché.
alpha_{D} = +98° ± 2° (c = 1 %).

### PREPARATION 1 : [1R-(1alpha,3béta)]-3-[(Z)-2-bromo 2-(4-chlorophényl) éthényl] 2,2-diméthyl cyclopropanecarboxylate de méthyle et isomère 1R-[1alpha,3béta(E)] correspondant

On dissout 7,8 g de (1R trans) 3-formyl 2,2-diméthyl cyclopropanecarboxylate de méthyle, 17,7 g de [bromo (4-chlorophényl) méthyl] phosphonate de diméthyle dans 70 cm³ de tétrahydrofuranne. On refroidit à -40°C et ajoute en 45 minutes 6,16 g de terbutyle de potassium en solution dans 60 cm³ de tétrahydrofuranne. On agite la solution ainsi obtenue pendant 1 heure à -40°/-45°C. On verse sur une solution aqueuse saturée de phosphate acide de sodium, extrait à l'éther isopropylique, lave, sèche et amène à sec. On obtient ainsi 18,7 g de produit que l'on chromatographie sur silice en éluant à l'aide du mélange hexane-éther isopropylique (95-5). On obtient 2,97 g d'isomère Z (17 %).

| Spectre RMN CDCl₃ 60 MHz : | |
|---|---|
| protons aromatiques | 7,16 à 7,68 ppm |
| proton vinylique | 5,87 - 6 ppm |
| protons des méthyles géminés | 1,23 - 1,35 ppm |
| protons de CO₂-CH₃ | 3,7 ppm |
| protons en 1 du cyclopropane | 1,63 - 1,73 ppm |
| protons en 3 du cyclopropane | 2,35 - 2,56 ppm |
| On obtient d'autre part 5,91 g d'isomère (34,5 %). | |

| Spectre RMN CDCl₃ 60 MHz : | |
|---|---|
| protons aromatiques | 7,32 ppm |
| proton vinylique | 5,85 - 5,98 ppm |
| protons des méthyles géminés | 1,17 - 1,22 ppm |
| protons en 1 du cyclopropane | 1,52 ppm |
| protons en 3 du cyclopropane | 1,87 - 2,1 ppm |

### EXEMPLE 2 : [1R-[1alpha(S*),3béta]]-3-[(Z)-2-(4-chlorophényl) 3,3,3-trifluoro 1-propényl] 2,2-diméthyl cyclopropanecarboxylate de cyano (4-fluoro 3-phénoxy phényl) méthyle

### STADE A : [1R-(1alpha,3béta)]-3-[(Z)-2-(4-chlorophényl) 3,3,3-trifluoro 1-propényl] 2,2-diméthyl cyclopropanecarboxylate de 2-(triméthylsilyl) éthyle

On dissout 3,06 g de [1R-(1alpha,3béta)]-3-[(Z)-2-bromo 2-(chlorophényl) éthényl] 2,2-diméthyl cyclopropanecarboxylate de 2-(triméthyl silyl) éthyle (préparation 2) et 3,86 g de trifluoroacétate de sodium dans 30 cm³ de N-méthyl pyrrolidone. On ajoute 2,7 g d'iodure de cuivre. On maitient le mélange réactionnel pendant 6 heures à 160°C. On laisse revenir à la température ambiante, filtre, rince, élimine les impuretés, extrait à l'éther isopropylique, dilue à l'hexane, lave à l'aide d'une solution saturée de chlorure de sodium, sèche sur sulfate de sodium et amène à sec. On récupère 3,3 g de produit que l'on chromatographie sur silice en éluant par le mélange hexane-toluène (6-4). On isole 1,69 g de produit recherché que l'on rechromatographie en éluant à l'aide du mélange hexane-éther isopropylique (95-5). On obtient ainsi 1,35 g de produit attendu.

| Spectre RMN CDCl₃ ppm : | |
|---|---|
| protons aromatiques | 7,08 à 7,42 ppm |
| proton vinylique | 5,62 - 5,8 ppm |
| protons des méthyles géminés | 1,22 - 1,32 ppm |
| protons du carbone en 1 du cyclopropane | 1,62 - 1,72 ppm |
| protons du carbone en 3 du cyclopropane | 2,35 à 2,82 ppm |
| protons en alpha du CO₂ | 4,05 à 4,33 ppm |
| protons en béta du CO₂ | 0,86 à 1,13 ppm |

### STADE B : [1R-[1alpha(S*),3béta]]-3-[(Z)-2-(4-chlorophényl) 3,3,3-trifluoro 1-propényl] 2,2-diméthyl cyclopropanecarboxylate de cyano (4-fluoro 3-phénoxy phényl) méthyle

On agite pendant 6 heures à la température ambiante 1,32 g de [1R-(1alpha,3béta)]-3-[(Z)-2-(4-chlorophényl) 3,3,3-trifluoro 1-propényl] 2,2-diméthyl cyclopropanecarboxylate de 2-(triméthylsilyl) éthyle, et 6,3 cm³ d'une solution molaire de fluorure de tétrabutylammonium dans le tétrahydrofuranne. On verse sur un mélange d'eau et de glace, ajoute 1,55 cm³ d'une solution normale d'acide chlorhydrique, extrait à l'éther isopropylique, lave à l'aide d'une solution saturée de chlorure de sodium, sèche et amène à sec. On obtient 1,1 g de produit que l'on ajoute avec 780 mg d'alcool (S)-alpha cyano 4-fluoro 3-phénoxy benzyle dans 6 cm³ de chlorure de méthylène. On refroidit au bain de glace et ajoute une solution renfermant 650 mg de dicyclohexylcarbodiimide, 10 mg de 4-diméthylaminopyridine et 4 cm³ de chlorure de méthylène. On agite pendant 5 minutes à 0° ± 5°C, puis 1 heure à la température ambiante. On filtre et rince à l'éther isopropylique. On récupère ainsi 1,7 g de produit que l'on chromatographie sur silice en éluant avec le mélange hexane-éther isopropylique (8-2). On obtient 416 g de produit recherché fondant à 78°C.
alpha_{D} = +49° ± 1,5° (c = 1 % CHCl₃).

### PREPARATION 2 : [1R-(1alpha,3béta)]-3-[(Z)-2-bromo 2-(4-chlorophényl) éthényl] 2,2-diméthyl cyclopropanecarboxylate de 2-(triméthyl silyl) éthyle et isomère E correspondants

On dissout 2,84 g d'acide (1R trans) 2,2-diméthyl 3-formyl cyclopropanecarboxylique, 7,5 g de [bromo (4-chlorophényl) méthyl] phosphonate de diméthyle dans 20 cm³ de tétrahydrofuranne. On refroidit à -40°/-45°C et ajoute en 25 minutes une solution de 5 g de terbutylate de potassium dans 25 cm³ de tétrahydrofurane. On agite la solution obtenue pendant 1 heure à -40°/-45°C. On verse dans une solution aqueuse saturée de phosphate acide de sodium, extrait à l'éther isopropylique, lave, sèche et amène à sec. On obtient 7,3 g de résidu que l'on dissout dans 20 cm³ de chlorure de méthyle. On ajoute 2,85 cm³ de triméthylsilyléthanol. On ajoute ensuite à 0°C environ 4,53 g de dicyclohexylcarbodiimide, 70 mg de 4-diméthylaminopyridine et 15 cm³ de chlorure de méthylène. On agite 5 minutes au bain de glace, puis une heure à la température ambiante. On filtre, rince à l'éther isopropylique et amène à sec. On obtient 9 g de produit que l'on chromatographie à l'aide du mélange hexane-éther isopropylique (95-5). On obtient 3,11 g d'isomère Z.

| Spectre RMN CDCl₃ : | |
|---|---|
| protons aromatiques | 7,15 - 7,52 ppm |
| protons des méthyles géminés | 1,24 - 1,37 ppm |
| proton vinylique | 5,87 - 6 ppm |
| protons du carbone en 3 du cyclopropane | 2,33 - 2,55 ppm |
| protons du carbone en 1 du cyclopropane | 1,60 1,7 ppm |
| protons en alpha du CO₂ | 4,05 - 4,33 ppm |
| protons en béta du CO₂ | 0,86 à 1,15 ppm |
| On obtient également 1,88 g d'isomère E | |
| protons aromatiques | 7,32 ppm |
| protons des méthyles géminés | 1,17 1,22 ppm |
| proton vinylique | 5,87 - 6 ppm |
| protons du carbone en 3 du cyclopropane | 1,85 à 2,08 ppm |
| protons du carbone en 1 du cyclopropane | 1,47 - 1,57 ppm |
| protons en alpha du CO₂ | 3,96 - 4,25 ppm |
| protons en béta du CO₂ | 0,8 - 1,08 ppm |

### EXEMPLE 3 : [1R-[1alpha(RS),3béta]]-3-[(Z)-2-(4-chlorophényl) 3,3,3-trifluoro 1-propényl] cyclopropanecarboxylate de 1-(6-phénoxy 2-pyridyl) éthyle

En opérant comme à l'exemple 1, mais en utilisant au stade B 1,07 g de (RS)-6-(3-phénoxy) alpha-méthyl 2-pyridine méthanol, on obtient 3 g de produit que l'on chromatographie sur silice (éluant : hexane-éther isopropylique (9-1)). On recueille 1,97 g de produit attendu.

### EXEMPLE 4 : Préparation d'un concentré soluble

On effectue un mélange homogène de :

| | |
|---|---|
| Produit de l'exemple 1 | 0,25 g |
| Butoxyde de pipéronyle | 1,00 g |
| Tween 80 | 0,25 g |
| Topanol A | 0,1 g |
| Eau | 98,4 g |

### EXEMPLE 5 : Préparation d'un concentré émulsifiable

On mélange intimement :

| | |
|---|---|
| Produit de l'exemple 2 | 0,015 g |
| Butoxyde de pipéronyle | 0,5 g |
| Topanol A | 0,1 g |
| Tween 80 | 3,5 g |
| Xylène | 95,885 g |

### EXEMPLE 6 : Préparation d'un concentré émulsifiable

On effectue un mélange homogène de :

| | |
|---|---|
| Produit de l'exemple 1 | 1,5 g |
| Tween 80 | 20,0 g |
| Topanol A | 0,1 g |
| Xylène | 78,4 g |

### EXEMPLE 7 : Préparation d'une composition fumigène

On mélange d'une façon homogène :

| | |
|---|---|
| Produit de l'exemple 1 | 0,25 g |
| Poudre de Tabu | 25,00 g |
| Poudre de feuille de cèdre | 40,00 g |
| Poudre de bois de pin | 33,75 g |
| Vert brillant | 0,50 g |
| p-nitrophénol | 0,50 g |

### EXEMPLE 8 : Exemples de compositions

On a préparé des compositions répondant à la formule suivante :

| | |
|---|---|
| Produit de l'exemple 1 | 1700,00 g |
| Diméthylformamide | 40,00 cc |
| Huile d'olive | 40,00 cc |

### EXEMPLE 9 : Préparation d'un concentré émulsifiable

On mélange intimement :

| | |
|---|---|
| Produit de l'exemple 1 | 0,015 g |
| Butoxyde de pipéronyle | 0,5 g |
| Topanol A | 0,1 g |
| Xylène | 99,385 g |

### EXEMPLE 10 : Préparation d'un concentré émulsifiable

On effectue un mélange homogène de :

| | |
|---|---|
| Produit de l'exemple 2 | 1,5 g |
| Tween 80 | 20,0 g |
| Topanol A | 0,1 g |
| Xylène | 78,4 g |

### ETUDE BIOLOGIQUE

### 1) Etude de l'activité létale sur mouche domestique

Les insectes tests sont des mouches domestiques femelles âgées de 4 jours. On opère par application topique de 1 µl de solution acétonique sur le thorax dorsal des insectes à l'aide du micromanipulateur d'ARNOLD. On utilise 50 individus par traitement. On effectue le contrôle de mortalité vingt-quatre heures après traitement.

Les essais sont effectués sans synergiste ou avec addition de butoxyde de pipéronyle (10 parties de synergiste pour 1 partie de composé à tester). Les résultats expérimentaux, résumés dans le tableau suivant, sont exprimés en DL₅₀ ou dose (en nanogrammes) nécessaire pour tuer 50 % des insectes :

| Composé de l'exemple | DL₅₀ en ng/insecte |
|---|---|
| 1 | 19,6 |
| 2 | 5,3 |

### 2) Etude de l'effet létal sur Aphis cracivora

On utilise des adultes après 7 jours et l'on emploie 10 Aphis par concentration utilisée. On utilise une méthode de contact-injection. On effectue le traitement au pistolet de Fisher d'une feuille de fève que l'on dépose dans une boîte de Pétri en matière plastique sur une rondelle de papier humidifiée. Le traitement est effectué à l'aide de 2 ml de solution acétonique de produit à tester (1 ml par face de feuille). L'infestation par les insectes est effectuée après séchage de la feuille. On maintient les insectes en contact avec la feuille pendant une heure. On place les insectes sur des feuilles non traitées et contrôle la mortalité au bout de 24 heures.

Les résultats expérimentaux obtenus sont résumés dans le tableau suivant :

| Composé de l'exemple | CL₅₀ en mg/l |
|---|---|
| 1 | 1,34 |
| 2 | 0,6 |

### 3) Etude de l'activité acaricide chez les animaux

### a) Etude de l'activité sur larve de Boophilus Microplus :

La substance à tester est dissoute dans un mélange constitué de diméthylformamide, d'émulsifiants et d'Arcopal de façon à obtenir un concentré émulsifiant à 10 %. On dilue ce concentré avec de l'eau pour obtenir des solutions de concentration souhaitée de 100, 10 et 1 ppm.

Au moyen d'une tour à pulvérisation, on pulvérise les différentes solutions ci-dessus sur des larves de tiques de boeufs des tropiques, de type Boophilus Microplus et on détermine après 24 heures, par comptage des larves mortes et vivantes, le pourcentage de mortalité.

Les résultats sont les suivants :

| Doses en ppm | Résultats % de mortalité Produit des exemples | | |
|---|---|---|---|
| | 1 | 2 | 3 |
| 100 | 100 | 100 | - |
| 10 | 100 | 100 | 100 |
| 1 | 100 | 100 | 100 |

Conclusion : les produits des exemples 1, 2 et 3 présentent une activité remarquable.

### b) Etude de l'activité sur l'inhibition de la reproduction de tiques

### Boophilus Microplus

On plonge des femelles de Boophilus Microplus prêtes à pondre, pendant 5 minutes, dans les solutions préparées ci-dessus, puis on les porte dans une enceinte chauffée pour la ponte.

On détermine :
a - le pourcentage de tiques n'ayant pas pondu,
b - la quantité d'oeufs pondus en fonction d'un témoin,
c - le pourcentage de larves ayant éclos.

On calcule, en fonction des chiffres obtenus, le pourcentage d'inhibition de la reproduction, 100 % signifie que l'inhibition est totale et 0 % que la reproduction est identique à celle obtenue avec les témoins. On obtient

| Doses en ppm | Résultats Produit | % d'inhibition des exemples | |
|---|---|---|---|
| | 1 | 2 | 3 |
| 100 | 100 | 100 | 100 |
| 50 | 100 | 100 | 100 |
| 25 | 100 | 100 | 100 |
| 12,5 | 100 | 100 | 100 |
| 6,2 | 100 | 100 | 100 |
| 3,1 | 100 | 100 | 100 |
| 1,5 | 100 | 100 | 100 |
| 0,75 | 100 | 100 | 100 |
| 0,38 | 100 | 100 | 85 |
| 0,19 | 100 | 100 | - |

Conclusion : les produits des exemples 1, 2 et 3 présentent une activité remarquable.

## Revendications

1. Les produits de formule (A) : 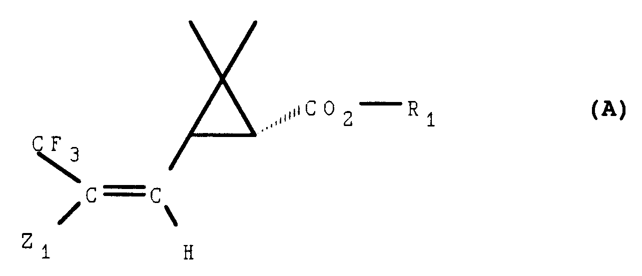 dans laquelle la copule cyclopropanique est de structure (1R, trans), la géométrie de la double liaison est Z,
Z₁ représente un radical aryle éventuellement substitué par un atome d'halogène, R₁ représente un des groupes : 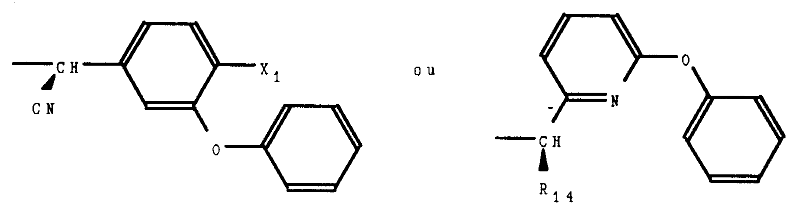 dans lesquels X₁ représente un atome d'hydrogène ou de fluor et R₁₄ représente un atome d'hydrogène ou un des radicaux méthyle, éthynyle ou cyano.

2. Les produits de formule (A) telle que définie à la revendication 1 dans laquelle Z₁ représente le radical 4-chloro phényle.

3. Le produit de formule (A₁) :

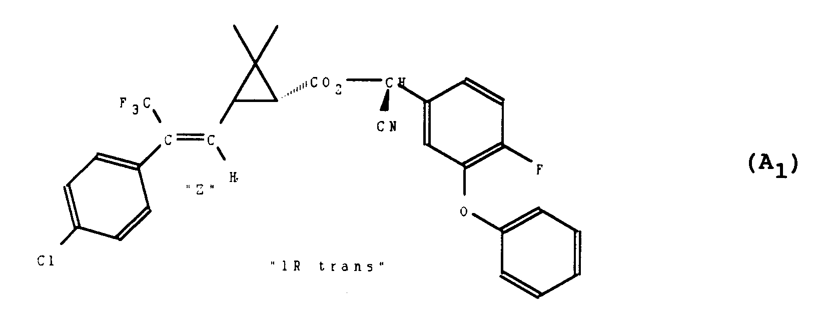

4. Le produit selon la revendication 3 dans laquelle le carbone chiral du groupe cyano (4-fluoro 3-phénoxy phenyl) méthyle est de configuration S.

5. Le produit de formule (B₁) :

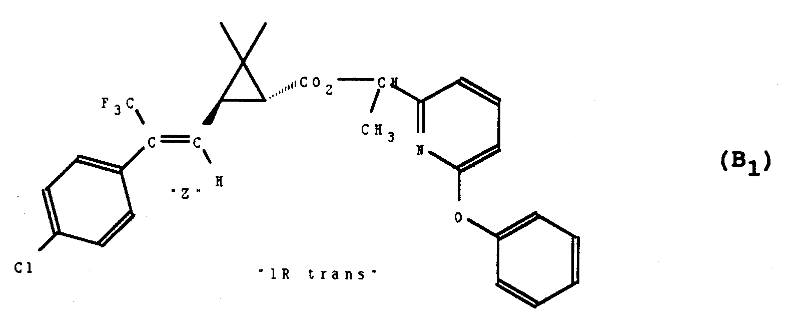

6. Le produit de formule (B₁) telle que définie à la revendication 5, dans laquelle le carbone chiral du groupe 1-(6-phénoxy 2-pyridyl) éthyle est de configuration R ou RS.

7. Procédé de préparation du produit de formule (A₁) telle que définie à la revendication 4, dans laquelle le carbone chiral du groupe cyano (4-fluoro 3-phénoxy phenyl) méthyle est de configuration S, caractérisé en ce que
a) on prépare un composé intermédiaire par réaction du [1R-(1alpha,3béta)]-3-[(Z)-2-(4-chlorophényl) 3,3,3-trifluoro 1-propényl] 2,2-diméthyl cyclopropanecarboxylate de 2-(triméthylsilyl) éthyle, et du fluorure de tétrabutylammonium,
c) on ajoute ce produit intermédiaire, à de l'alcool (S)-alpha cyano 4-fluoro 3-phénoxy benzyle dans du chlorure de méthylène, en présence de dicyclohexylcarbodiimide, et de 4-diméthylaminopyridine.

8. Procédé de préparation du produit de formule (B₁) telle que définie à la revendication 6, dans laquelle le carbone chiral du groupe 1-(6-phénoxy 2-pyridyl) éthyle est de configuration R, caractérisé en ce que
a) le [1R-(1alpha,3béta)]-3-[(Z)-2-(4-chlorophényl) 3,3,3-trifluoro 1-propényl] 2,2-diméthyl cyclopropanecarboxylate de méthyle est traité à la soude,
b) le produit ainsi obtenu et isolé est mis à réagir avec du (R)-6-(3-phénoxy) alpha-méthyl 2-pyridine méthanol en présence de dicyclohexylcarbodiimide, et de 4-diméthylaminopyridine .

9. Procédé selon la revendication 8, de préparation du produit de formule (B₁) telle que définie à la revendication 6, dans laquelle le carbone chiral du groupe 1-(6-phénoxy 2-pyridyl) éthyle est de configuration RS, en utilisant le (RS)-6-(3-phénoxy) alpha-méthyl 2-pyridineméthanol.

10. Application des composés de formule (A) tels que définis à l'une quelconque des revendications 1 à 6 à la lutte contre les parasites des végétaux, les parasites des locaux et les parasites des animaux à sang chaud.

11. Les compositions destinées à la lutte contre les parasites des végétaux, les parasites des locaux et les parasites des animaux à sang chaud, caractérisées en ce qu'elles renferment comme principe actif au moins un des produits définis à l'une quelconque des revendications 1 à 6.

12. Les compositions insecticides renfermant comme principe actif au moins l'un des produits définis à l'une quelconque des revendications 1 à 6.

13. Les compositions acaricides renfermant comme principe actif au moins l'un des produits définis à l'une quelconque des revendications 1 à 6.

14. Les compositions nématicides renfermant comme principe actif au moins l'un des produits définis à l'une quelconque des revendications 1 à 6.

15. Les compositions acaricides destinées à la lutte contre les parasites des animaux à sang chaud, notamment contre les tiques et les gales, caractérisées en ce qu'elles renferment comme principe actif au moins l'un des produits définis à l'une quelconque des revendications 1 à 6.

16. Les compositions destinées à l'alimentation animale renfermant comme principe actif au moins l'un des produits définis à l'une quelconque des revendications 1 à 6.

17. Associations douées d'activité insecticide, acaricide ou nématicide, caractérisées en ce qu'elles contiennent comme matière active, d'une part un au moins des composés de formule générale (A) telle que définie à la revendication 1, et d'autre part, un au moins des esters pyréthrinoïdes choisis dans le groupe constitué par les esters d'alléthrolones, d'alcool 3,4,5,6-tétrahydrophtalimido méthylique, d'alcool (5-benzyl 3-furyl) méthylique, d'alcool 3-phénoxy benzylique et d'alcools alpha-cyano 3-phénoxy benzyliques des acides chrysanthémiques, par les esters d'alcool (5-benzyl 3-furyl) méthylique des acides 2,2-diméthyl 3-(2-oxo 3-tétrahydrothiophénylidène méthyl) cyclopropanecarboxyliques, par les esters d'alcool 3-phénoxy benzylique et d'alcools alpha-cyano 3-phénoxy benzyliques des acides 2,2-diméthyl 3-(2,2-dichlorovinyl) cyclopropanecarboxyliques, par les esters d'alcool alpha-cyano 3-phénoxy benzyliques d'acides 2,2-diméthyl 3-(2,2-dibromovinyl) cyclopropanecarboxyliques, par les esters d'alcool 3-phénoxy benzylique des acides 2-parachlorophényl 2-isopropyl acétiques, par les esters d'alléthrolones d'alcool 3,4,5,6-tétrahydrophtalimido-méthylique, d'alcool (5-benzyl 3-furyl) méthylique, d'alcool 3-phénoxy benzylique et d'alcools alpha-cyano 3-phénoxy benzyliques des acides 2,2-diméthyl 3-(1,2,2,2-tétrahalo éthyl) cyclopropanecarboxyliques, dans lesquels "halo" représente un atome de fluor, de chlore ou de brome.

18. Les compositions telles que définies à l'une des revendications 11 à 17, caractérisées en ce qu'elles renferment en outre un synergiste des pyréthrinoïdes.

19. Les produits intermédiaires de formule (I), dont les noms suivent :
- le [1R-(1alpha,3béta)]-3-[(Z)-2-(4-chlorophényl) 3,3,3-trifluoro 1-propényl] 2,2-diméthyl cyclopropanecarboxylate de méthyle,
- le [1R-(1alpha,3béta)]-3-[(Z)-2-(4-chlorophényl) 3,3,3-trifluoro 1-propényl] 2,2-diméthyl cyclopropanecarboxylate de 2-(triméthylsilyl) éthyle.

## Claims

1. The products of general formula (A): 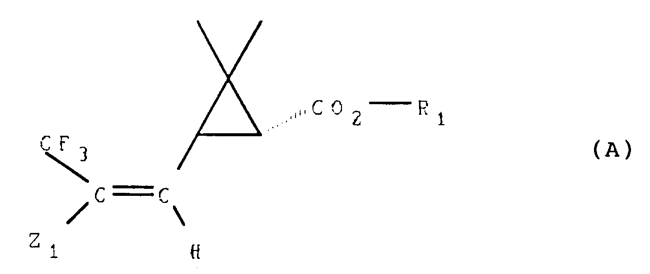 in which the cyclopropane copula is of (1R, trans) structure, the geometry of the double bond is Z,
Z₁ represents an aryl radical optionally substituted by a halogen atom, R₁ represents one of the groups: 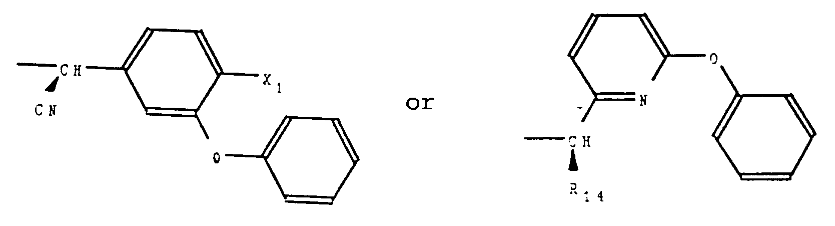 in which X₁ represents a hydrogen or fluorine atom and R₁₄ represents a hydrogen atom or one of the methyl, ethynyl or cyano radicals.

2. The products of formula (A) as defined in claim 1 in which Z₁ represents the 4-chloro phenyl radical.

3. The product of formula (A₁):

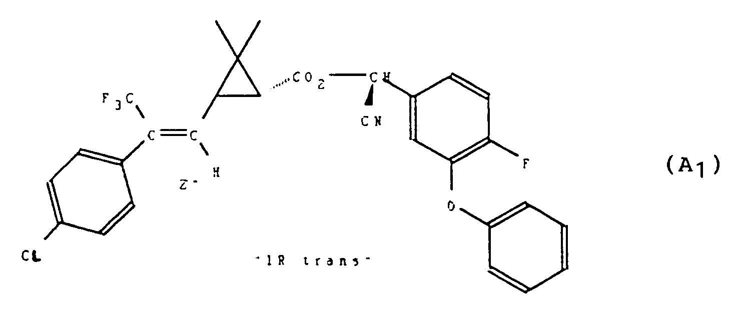

4. The product according to claim 3 in which the chiral carbon of the cyano (4-fluoro 3-phenoxy phenyl) methyl group is of S configuration.

5. The product of formula (B₁):

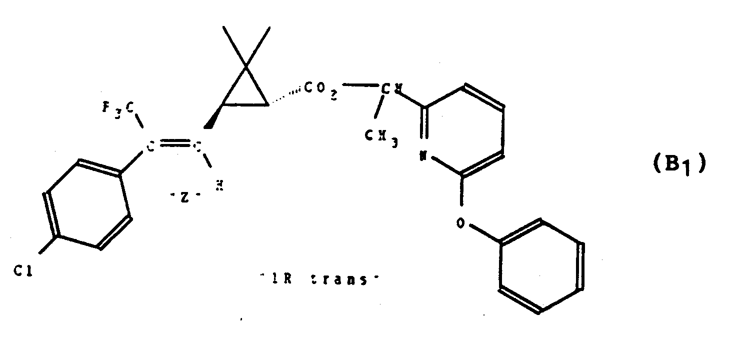

6. The product of formula (B₁) as defined in claim 5, in which the chiral carbon of the 1-(6-phenoxy 2-pyridyl) ethyl group is of R or RS configuration.

7. Preparation process for the product of formula (A₁) as defined in claim 4, in which the chiral carbon of the cyano (4-fluoro 3-phenoxy phenyl) methyl group is of S configuration, characterized in that
a) an intermediate compound is prepared by reacting 2-(trimethylsilyl) ethyl [1R-(1alpha,3beta)]-3-[(Z)-2-(4-chlorophenyl) 3,3,3-trifluoro 1-propenyl] 2,2-dimethyl cyclopropanecarboxylate and tetrabutylammonium fluoride,
c) this intermediate product is added to (S)-alpha cyano 4-fluoro 3-phenoxy benzyl alcohol in methylene chloride, in the presence of dicyclohexylcarbodiimide, and 4-dimethylaminopyridine.

8. Preparation process for the product of formula (B₁) as defined in claim 6, in which the chiral carbon of the 1-(6-phenoxy 2-pyridyl) ethyl group is of R configuration, characterized in that
a) methyl [1R-(1alpha,3beta)]-3-[(Z)-2-(4-chlorophenyl) 3,3,3-trifluoro 1-propenyl] 2,2-dimethyl cyclopropanecarboxylate is treated with soda,
b) the product thus obtained and isolated is reacted with (R)-6-(3-phenoxy) alpha-methyl 2-pyridine methanol in the presence of dicyclohexylcarbodiimide, and 4-dimethylaminopyridine.

9. Process according to claim 8, for the preparation of the product of formula (B₁) as defined in claim 6, in which the chiral carbon of the 1-(6-phenoxy 2-pyridyl) ethyl group is of RS configuration, using (RS)-6-(3-phenoxy) alpha-methyl 2-pyridinemethanol.

10. Use of the compounds of formula (A) as defined in any one of claims 1 to 6 for combating parasites of vegetation, parasites in premises and parasites of warm-blooded animals.

11. The compositions intended for combating parasites of vegetation, parasites in premises and parasites of warm-blooded animals, characterized in that they contain as active ingredient at least one of the products defined in any one of claims 1 to 6.

12. The insecticide compositions containing as active ingredient at least one of the products defined in any one of claims 1 to 6.

13. The acaricide compositions containing as active ingredient at least one of the products defined in any one of claims 1 to 6.

14. The nematicide compositions containing as active ingredient at least one of the products defined in any one of claims 1 to 6.

15. The acaricide compositions intended for combating parasites of warm-blooded animals, in particular ticks and mites, characterized in that they contain as active ingredient at least one of the products defined in any one of claims 1 to 6.

16. The compositions intended for animal fodder containing as active ingredient at least one of the products defined in any one of claims 1 to 6.

17. Combinations endowed with insecticide, acaricide or nematicide activity, characterized in that they contain as active ingredient, on the one hand at least one of the compounds of general formula (A) as defined in claim 1, and on the other hand, at least one of the pyrethrinoid esters chosen from the group constituted by the esters of allethrolones, of 3,4,5,6-tetrahydrophthalimidomethyl alcohol, of (5-benzyl-3-furyl) methyl alcohol, of 3-phenoxy benzyl alcohol and of alpha-cyano-3-phenoxy benzyl alcohols with chrysanthemic acids, by the esters of (5-benzyl 3-furyl) methyl alcohol with 2,2-dimethyl 3-(2-oxo 3-tetrahydrothiophenylidene methyl) cyclopropanecarboxylic acids, by the esters of 3-phenoxy benzyl alcohol and of alpha-cyano 3-phenoxy benzyl alcohols with 2,2-dimethyl 3-(2,2-dichlorovinyl) cyclopropanecarboxylic acids, by the esters of alpha-cyano 3-phenoxy benzyl alcohol with 2,2-dimethyl 3-(2,2-dibromovinyl) cyclopropanecarboxylic acids, by the esters of 3-phenoxy benzyl alcohol with 2-parachlorophenyl 2-isopropyl acetic acids, by the esters of allethrolones, of 3,4,5,6-tetrahydrophthalimido-methyl alcohol, of (5-benzyl 3-furyl) methyl alcohol, of 3-phenoxy benzyl alcohol, and of alpha-cyano 3-phenoxybenzyl alcohols with 2,2-dimethyl 3-(1,2,2,2-tetrahalo ethyl) cyclopropanecarboxylic acids, in which "halo" represents a fluorine, chlorine or bromine atom.

18. The compositions as defined in one of claims 11 to 17, characterized in that they contain in addition a pyrethrinoid synergist.

19. The intermediate products of formula (I), of which the names follow:
- methyl [1R-(1alpha,3beta)]-3-[(Z)-2-(4-chlorophenyl) 3,3,3-trifluoro 1-propenyl] 2,2-dimethyl cyclopropanecarboxylate,
- 2-(trimethylsilyl) ethyl [1R-(1alpha,3beta)]-3-[(Z)-2-(4-chlorophenyl) 3,3,3-trifluoro 1-propenyl] 2,2-dimethyl cyclopropanecarboxylate.

## Patentansprüche

1. Die Produkte der Formel (A) 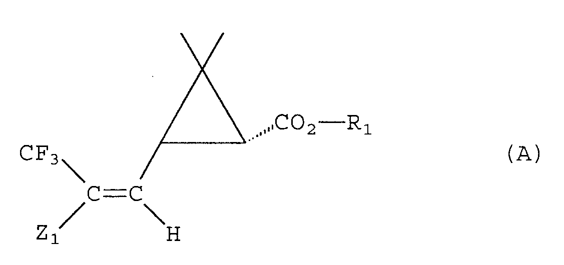 in der die Cyclopropan-Kopplung die Struktur (1R, trans) besitzt, die Geometrie der Doppelbindung Z ist,
Z₁ einen Arylrest darstellt, gegebenenfalls substituiert durch ein Halogenatom, und R₁ eine der Gruppen 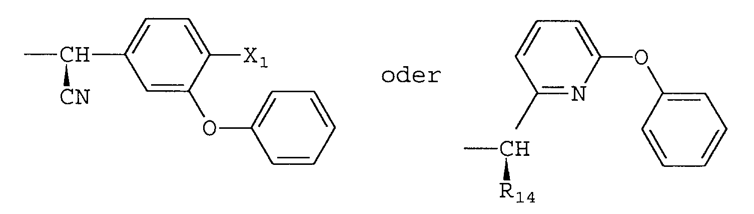 bedeutet, worin X₁ ein Wasserstoff- oder Fluoratom und R₁₄ ein Wasserstoffatom oder einen der Reste Methyl, Ethinyl oder Cyano darstellen.

2. Die Produkte der Formel (A) wie in Anspruch 1 definiert, worin Z₁ den 4-Chlorphenylrest darstellt.

3. Das Produkt der Formel (A₁)

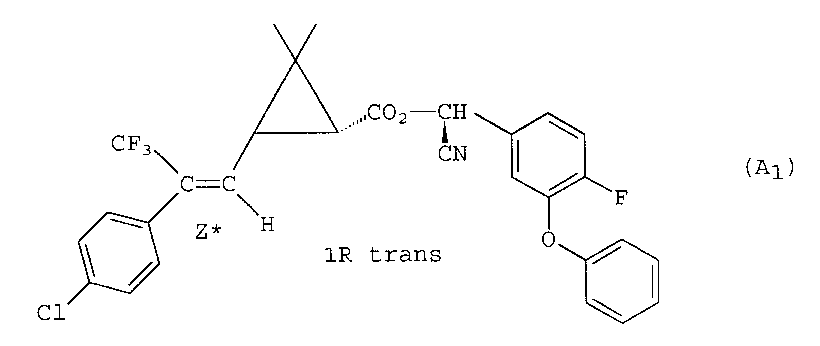

4. Das Produkt nach Anspruch 3, worin der chirale Kohlenstoff der Gruppe Cyano-(4-fluor-3-phenoxyphenyl)-methyl die Konfiguration S besitzt.

5. Das Produkt der Formel (B₁)

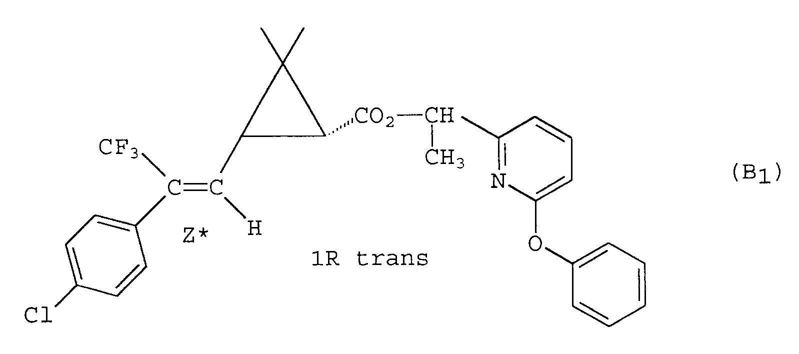

6. Das Produkt der Formel (B₁) wie in Anspruch 5 definiert, worin der chirale Kohlenstoff der Gruppe 1-(6-Phenoxy-2-pyridyl)-ethyl die Konfiguration R oder RS besitzt.

7. Verfahren zur Herstellung des Produktes der Formel (A₁) wie in Anspruch 4 definiert, worin der chirale Kohlenstoff der Gruppe Cyano-(4-fluor-3-phenoxyphenyl)-methyl die Konfiguration S besitzt, dadurch gekennzeichnet, daß man
a) eine Zwischenverbindung herstellt durch Reaktion von [1R-(1α, 3β)]-3-[(Z)-2-(4-Chlorphenyl)-3,3,3-trifluor-1-propenyl]-2,2-dimethyl-cyclopropancarbonsäure-2-(trimethylsilyl)-ethylester und Tetrabutylammoniumfluorid, und
b) dieses Zwischenprodukt zu dem (S)-α-Cyano-4-fluor-3-phenoxybenzylalkohol in Methylenchlorid gibt, in Anwesenheit von Dicyclohexylcarbodiimid und 4-Dimethylaminopyridin.

8. Verfahren zur Herstellung des Produktes (B₁) wie in Anspruch 6 definiert, worin der chirale Kohlenstoff der Gruppe 1-(6-Phenoxy-2-pyridyl)-ethyl die Konfiguration R besitzt, dadurch gekennzeichnet, daß
a) der [1R-(1α,3β)]-3-[(Z)-2-(4-Chlorphenyl)-3,3,3-trifluor-1-propenyl]-2,2-dimethyl-cyclopropancarbonsäure-methylester mit Natriumhydroxid behandelt wird, und
b) das auf diese Weise erhaltene Produkt isoliert und mit (R)-6-(3-Phenoxy)-α-methyl-2-pyridin-methanol in Anwesenheit von Dicyclohexylcarbodiimid und 4-Dimethylaminopyridin zur Reaktion gebracht wird.

9. Verfahren nach Anspruch 8 zur Herstellung des Produktes der Formel (B₁) wie in Anspruch 6 definiert, worin der chirale Kohlenstoff der Gruppe 1-(6-Phenoxy-2-pyridyl)-ethyl die Konfiguration RS besitzt, unter Verwendung von (RS)-6-(3-Phenoxy)-α-methyl-2-pyridin-methanol.

10. Verwendung der Verbindungen der Formel (A) wie in irgendeinem der Ansprüche 1 bis 6 definiert zur Bekämpfung von Parasiten an Pflanzen, Parasiten in Räumen und Parasiten an Warmblüter-Tieren.

11. Zusammensetzungen für die Bekämpfung von Parasiten an Pflanzen, Parasiten in Räumen und Parasiten an Warmblüter-Tieren, dadurch gekennzeichnet, daß sie als Wirkstoff mindestens eines der in irgendeinem der Ansprüche 1 bis 6 definierten Produkte umfassen.

12. Insektizide Zusammensetzungen, umfassend als Wirkstoff mindestens eines der in irgendeinem der Ansprüche 1 bis 6 definierten Produkte.

13. Akarizide Zusammensetzungen, umfassend als Wirkstoff mindestens eines der in irgendeinem der Ansprüche 1 bis 6 definierten Produkte.

14. Nematizide Zusammensetzungen, umfassend als Wirkstoff mindestens eines der in irgendeinem der Ansprüche 1 bis 6 definierten Produkte.

15. Akarizide Zusammensetzungen zur Bekämpfung von Parasiten an Warmblüter-Tieren, insbesondere von Zecken und Räude, dadurch gekennzeichnet, daß sie als Wirkstoff mindestens eines der in irgendeinem der Ansprüche 1 bis 6 definierten Produkte umfassen.

16. Zusammensetzungen für die Tierfütterung, umfassend als Wirkstoff mindestens eines der in irgendeinem der Ansprüche 1 bis 6 definierten Produkte.

17. Assoziationen mit insektizider, akarizider oder nematizider Aktivität, dadurch gekennzeichnet, daß sie als Wirkstoff einerseits mindestens eine der Verbindungen der Formel (A) wie in Anspruch 1 definiert, und andererseits mindestens einen Pyrethrinoid ester enthalten, ausgewählt aus der durch die Ester von Allethrolonen, 3,4,5,6-Tetrahydrophthalimid-methylalkohol, (5-Benzyl-3-furyl)-methylalkohol, 3-Phenoxy-benzylalkohol und den α-Cyano-3-phenoxybenzylalkoholen mit Chrysanthemsäuren, durch die Ester von (5-Benzyl-3-furyl)-methylalkohol mit den 2,2-Dimethyl-3-(2-oxo-3-tetrahydrothiophenyliden-methyl)-cyclopropancarbonsäuren, durch die Ester von 3-Phenoxy-benzylalkohol und den α-Cyano-3-phenoxybenzylalkoholen mit den 2,2-Dimethyl-3-(2,2-dichlorvinyl)-cyclopropancarbonsäuren, durch die Ester von α-Cyano-3-phenoxybenzylalkoholen mit den 2,2-Dimethyl-3-(2,2-dibromvinyl)-cyclopropancarbonsäuren, durch die Ester von 3-Phenoxy-benzylalkohol mit 2-para-Chlorphenyl-2-isopropyl-essigsäuren, und durch die Ester von Allethrolonen, 3,4,5,6-Tetrahydrophthalimid-methylalkohol, (5-Benzyl-3-furyl)-methylalkohol, 3-Phenoxy-benzylalkohol und den α-Cyano-3-phenoxybenzylalkoholen mit den 2,2-Dimethyl-3-(1,2,2,2-tetrahalogenethyl)-cyclopropancarbonsäuren, worin "Halogen" ein Fluor-, Chlor- oder Bromatom darstellt, gebildeten Gruppe.

18. Zusammensetzungen wie in einem der Ansprüche 11 bis 17 definiert, dadurch gekennzeichnet, daß sie außerdem einen Synergisten der Pyrethrinoide umfassen.

19. Die Zwischenprodukte der Formel (I), deren Namen folgen:
- [1R-(1α,3β)]-3-[(Z)-2-(4-Chlorphenyl)-3,3,3-trifluor-1-propenyl]-2,2-dimethyl-cyclopropancarbonsäure-methylester,
- [1R-(1α, 3β)]-3-[(Z)-2-(4-Chlorphenyl)-3,3,3-trifluor-1-propenyl]-2,2-dimethyl-cyclopropancarbonsäure-2-(trimethylsilyl)ethylester.
